(19) Europäisches Patentamt
European Patent Office
Office européen des brevets

(11) Veröffentlichungsnummer: **0 313 942 A1**

(12) # EUROPÄISCHE PATENTANMELDUNG

(21) Anmeldenummer: **88117157.3**

(22) Anmeldetag: **14.10.88**

(51) Int. Cl.[4]: **A61K 49/00 , A61K 43/00**

(30) Priorität: **15.10.87 SU 4313068**

(43) Veröffentlichungstag der Anmeldung: **03.05.89 Patentblatt 89/18**

(84) Benannte Vertragsstaaten: **CH DE FR GB IT LI SE**

(71) Anmelder: **MOSKOVSKY GOSUDARSTVENNY UNIVERSITET imeni M.V. LOMONOSOVA**
**Leninskie Gory**
**Moskau(SU)**

(72) Erfinder: **Dzbanovsky, Nikolai Nikolaevich**
**ulitsa V. Ulbrikhta, 8, kv.95**
**Moscow(SU)**
Erfinder: **Polsachev, Viktor Iosifovich**
**Izmailosvky prospekt, 123/1, kv.29**
**Moscow(SU)**
Erfinder: **Potemkina, Elena Vasilievna**
**Frunkzenskava naberezhnaya, 8, kv.26**
**Moscow(SU)**
Erfinder: **Rakhimov, Alexandr Tursunovich**
**Rostovskava naberezhnaya, 1, kv.95**
**Moscow(SU)**
Erfinder: **Rubin, Leonid Borisovich**
**Lomonosovsky prospekt, 14, kv.499**
**Moscow(SU)**
Erfinder: **Osipov, Alexandr Sergeevich**
**Mosfilmovskaya ulitsa, 32, kv.42**
**Moscow(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Verwendung von Fluoronderivaten und Mittel zur Kontrastierung von bösartigen Neubildungen bei deren Diagnostik.**

(57) Die Erfindung bezieht sich auf die Anwendung von Fluoronderivaten mit Ausnahme des Fluoreszeins und seiner Salze der allgemeinen Formel I in der Onkologie für die Diagnostik, darunter auch für die Frühdiagnostik von bösartigen Neubildungen, für die Bestimmung der Verbreitung einer bösartigen Schädigung, Bestimmung der Diagnose in Kliniken und Gesundheitsfürsorgestellen, die Überwachung der Behandlung von bösartigen Geschwulsten und die Feststellung von Nachoperationsrückfällen bei einer Röntgen-, Radioisotopen-, Fluoreszenz- und der NMR-Kontrastierung von bösartigen Neubildungen.

Im erfindungsgemäßen Mittel zur Kontrastierung können Fluoronderivate sowohl selbständig als auch in der Kombination mit Sacchariden und/oder Disacchariden und/oder Vitaminen und/oder Blockatoren der Durchdringungsfähigkeit der Zellmembranen zur Diagnostik angewendet werden.

EP 0 313 942 A1

# VERWENDUNG VON FLUORONDERIVATEN UND MITTEL ZUR KONTRASTIERUNG VON BÖSARTIGEN NEUBILDUNGEN BEI DEREN DIAGNOSTIK

Die vorliegende Erfindung bezieht sich auf die Medizin, und zwar auf die Onkologie.

Am effektivsten kann die vorliegende Erfindung für die Diagnostik, darunter auch für eine Frühdiagnostik von bösartigen Neubildungen, für die Bestimmung der Verbreitung der bösartigen Schädigung, die Bestimmung des Umfangs der Operationen, die Präzisierung der Diagnose in Kliniken und Gesundheitsfürsorgestellen, die Überwachung der Behandlung von bösartigen Geschwulsten und die Feststellung von Nachoperationsrückfällen angewendet werden.

Gegenwärtig stellt das Problem, bösartige Neubildungen, insbesondere in frühen Stadien, zu diagnostizieren, eines der aktuellsten Probleme in der Onkologie dar. Trotz einer breiten Anwendung von histologischen Methoden der Analyse, den großen Erfolgen der Ultraschall- und Röntgendiagnostik, der Endoskopieuntersuchungen sowie der Röntgen- und der NMR-Tomografie sind die Empfindliohkeit, die Genauigkeit und die Zugänglichkeit dieser Methoden nicht ausreichend. Eine der Richtungen für die Vervollkommnung der Diagnostik von bösartigen Geschwulsten besteht in der Anwendung von darin selektiv akkumulierten konstrastierenden Stoffen. Es ist z.B. gut bekannt, daß die bösartigen Geschwulste einige Farbstoffe in einer erhöhten Konzentration gegenüber den normalen Geweben akkumulieren, was im Prinzip für eine Fluoreszenzdiagnostik der Geschwulst nach einer charakterstischen Fluoreszenz des darin akkumulierten Farbstoffes angewendet werden könnte. Das für diese Zwecke verwendete Fluoreszein gab keine positiven Ergebnisse, da der Kontrast der Akkumulierung des Fluoreszeins im bösartigen Gewebe gegenüber dem normalen Gewebe für eine zuverlässige Diagnostik einer bösartigen Schädigung nicht ausreichend war (s. Ju.N. Efuni, Westnik otorinolaringologii, 1961, Nr. 2, S. 11-15).

Der Akkumulierungskontrast eines beliebigen fluoreszierenden kontrastierenden Stoffes in einer bösartigen Geschwulst wird im Vergleich zu einem normalen Gewebe durch das Verhältnis der Konzentrationen des darin enthaltenen Stoffes bestimmt.

Es ist ein Mittel zur Kontrastierung von bösartigen Geschwulsten bei der Diagnostik bekannt, welches aus einer wässerigen Lösung von Hämatoporphyrinderivaten besteht (s. Hematoporphyrin Derivative Photoradiation Therapy of Cancer, M., Berns Ed. Alan R. Liss Inc. NY 1984; G.H.M. Gijsbers, D. Breederveld, M.I.C. van Gemert, T.A. Boon, S. Langelaar, R.P.H. Rettschnick. Laser in the life sciences I, Nr. 1, 29-49, 1986).

Die Nachteile des Hämatoporphyrins als eines kontrastierenden Stoffes sind folgende: eine niedrige Selektivität der Akkumulierung in bösartigen Geweben gegenüber den normalen Geweben (der Akkumulierungskontrast beträgt etwa 4 relative Einheiten); die Notwendigkeit, diesen Stoff in hohen Konzentrationen anzuwenden; die Toxizität und das Vorhandensein negativer Nebeneffekte (z.B. eine fotodynamische Schädigung der Haut) auf den Organismus der Labortiere, der Zellkulturen und der Menschen.

Als einen weiteren Nachteil dieses Stoffes kann man bezeichnen, daß es damit unmöglich ist, Geschwulste der inneren Organe und Metastasen, z.B. in der Leber in Knochen und im Gehirn zu diagnostizieren, weil Hämatoporphyrin nur für eine Fluoreszenzkontrastierung der für eine optische Untersuchung zugänglichen Geschwulste, d.h. der Geschwulste, die entweder auf dem Körper selbst oder an der Innenfläche der Organe (Lungen, Verdauungssystem, Bauchhöhle u.a.) lokalisiert sind, angewendet werden kann.

Außerdem besteht der Nachteil dieses Stoffes darin, daß das Hämatopyrphyrin in seiner Zusammensetzung keine chemischen Elemente enthält, die radioaktive Isotope haben, was es unmöglich macht, diesen Stoff für eine Radioisotopendiagnostik anzuwenden.

Der vorliegenden Erfindung liegt die Aufgabe zugrunde, Stoffe zu finden, welche die oben genannten Nachteile bei der Kontrastierung bösartiger Neubildungen unabhängig von ihrer Lokalisation beseitigen.

Die gestellte Aufgabe wurde dadurch gelöst, daß ein Kreis von Derivaten der allgemeinen Formel I bestimmt wurde,

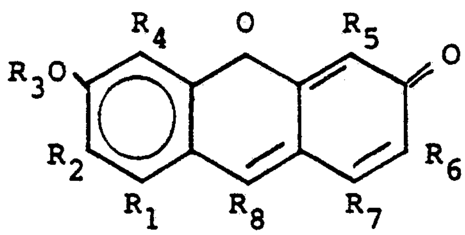

(I)

worin in beliebiger Kombination:

$R_1$ und $R_7$ in beliebiger Kombination Wasserstoff (H), Halogen (F, Cl, Br, J), $J^{131}$, $CH_3$, $CF_3$, $CCl_3$, $C_2H_5$, COOH, $COOCH_3$, $NO_2$, $NH_2$, OH, $OCH_3$, $OC_2H_5$ bedeuten;

$R_2$, $R_4$, $R_5$ und $R_6$ in beliebiger Kombination Wasserstoff (H), Halogen (F, Cl, Br, J), $J^{131}$, sowie die Substituenten, im weiteren als Gruppe A bezeichnet, und zwar aliphatischer $C_1$-$C_5$-Kohlenwasserstoff, linear oder verzweigt, einschließlich Alkyl, Alkenyl, Alkinyl in beliebiger Kombination von Einfach-, Doppel- oder Dreifachbindungen bedeuten; Gruppe B -Gruppe A, substituiert durch F, Cl, Br, J, $J^{131}$ von eins bis zur vollständigen Substitution bei beliebiger Kombination unter ihnen bedeutet;

$CH_3$, $CF_3$, $CBr_3$, $Cl_3$, $CJ_3^{131}$, COOH, COOA, COOB, OA, OB, $NO_2$, $NH_2$, eine Substituentengruppe, im weiteren als Gruppe W bezeichnet - Aryl (Phenyl, Naphtyl, Anthryl);

eine Substituentengruppe, im weiteren als Gruppe G bezeichnet, die Gruppe W, substituiert durch F, Cl, Br, J, $J^{131}$, $NO_2$, $NH_2$, OH, $HSO_3$, COOH, $COO^-$, NDE (die Bedeutung von D und E s. unten), NH- $\underset{\overset{\|}{O}}{C}$ -D, - $\underset{\overset{\|}{O}}{C}$ -D von einem bis zur vollständigen Substitution in beliebiger Kombination unter ihnen ist, worin die Gruppen, die im weiteren als D- und E-Gruppen bezeichnet werden, A, B, W, G, AB, AG, BW oder BG darstellen;

$R_3$ Wasserstoff (H), das Kation eines Alkalimetalls oder Ammonium, A, B, W, G; die Gruppe Q, und zwar Acyl (A-CH = O), die Gruppe Z, und zwar Acyl (B-CH = O) oder Glukuronsäure bedeutet;

$R_8$ Wasserstoff (H), A, B, W, G, AB, AG, BW, BG, die Gruppe 1-aliphatischer $C_1$-$C_{15}$-Kohlenwasserstoff, linear oder verzweigt, einschließlich Alkyl, Alkenyl, Alkinyl, in beliebiger Kombination von Einfach-, Doppel- und Dreifachbindungen bedeutet;

eine Gruppe K - die Gruppe I, substituiert durch F, Cl, Br, J, $J^{131}$, $NO_2$, $NH_2$, OH, $HSO_3$, COOH, $COO^-$ von eins bis zur vollständigen Substitution in beliebiger Kombination unter ihnen, AI, AK, BI, BK, WI, WK, GI, GK oder die Gruppe L -H- $\underset{\overset{\|}{S}}{C}$ -$NH_2$ ist,

die Gruppe M die Aminosäuren mit $C_1$-$C_5$, LM, Gruppe Y, die einen aromatischen Heterocyclus (Pyridyl, Chinolyl) bezeichnet, die Gruppe V, die die Gruppe Y ist, substituiert durch $NO_2$, $NH_2$, OH, COOH, $COO^-$, $HSO_3$, F, Cl, Br, J, $J^{131}$, COOA, COOB,

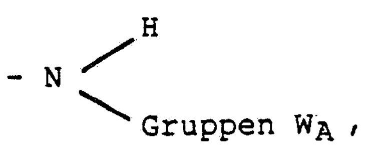

$CH_3$, $CF_3$, $CCl_3$, $CBr_3$, $CJ_3$, $CJ_3^{131}$ von eins bis zur vollständigen Substitution in beliebiger Kombination unter ihnen, AY, BY, AV, BV, WY, GY, GV, WV für eine Röntgen-, Radioisotopen-, Fluoreszenz- und NMR-Kontrastierung von bösartigen Neubildungen bedeutet.

Die Fluoronderivate der allgemeinen Formel I, worin $R_1$ und $R_7$ beide $J^{131}$ oder einer $J^{131}$ und der andere beliebig aus Wasserstoff (H), F, Cl, Br, J, $CH_3$, $CF_3$, $CCl_3$, $C_2H_5$, COOH, $COOCH_3$, $NO_2$, $NH_2$, OH, $OCH_3$ oder $OC_2H_2$ bedeuten,

$R_2$, $R_4$, $R_5$ und $R_6$ alle $J^{131}$ oder einer oder bis zu drei $J^{131}$ und die anderen eine oder mehrere aus der Gruppe Wasserstoff (H), F, Cl, Br, J, Gruppen A, B, W, G, AW, AG, BW, BG, $CH_3$, $CF_3$, $CCl_3$, $CBr_3$, $CJ_3$, $CJ_3^{131}$, COOH, COOA, COOB, OA, OB, $NO_2$ oder $NH_2$ bedeuten;

$R_3$ Wasserstoff (H) das Kation eines Alkalimetalls oder Ammonium, A, B, W, G, Q, Z oder Glukoronsäure bedeutet;

$R_8$ Wasserstoff (H) oder $J^{131}$ oder $J^{131}$ bedeutet, welches in beliebiger Kombination in die Gruppen A, B, W, AW, AG, BW, BG, I, K, AI, AK, BI, WI, GI, GK, L, M, LM, Y, V, AY, BY, BV, AV, GY, WV, GY, GV vorliegt, werden für eine Radioisotopenkontrastierung von bösartigen Neubildungen bevorzugt.

Fluoronderivate der allgemeinen Formel I, worin

$R_1$ und $R_7$ jeder aus der Gruppe Wasserstoff (H), $CH_3$, $NO_2$, $NH_2$, OH und $OCH_3$ ausgewählt ist,

$R_2$, $R_4$, $R_5$ und $R_6$ entweder alle $J^{131}$ oder eins bis drei $J^{131}$ und der Rest eins oder mehrere aus der Gruppe: Wasserstoff (H), $NO_2$, $NH_2$, $CH_3$, $CCl_3$, $CF_3$, $CJ_3$ und $CJ_3^{131}$ bedeuten;

$R_3$ Wasserstoff (H), Kation eines Alkalimetalls oder Ammonium bedeutet;

$R_8$ H oder $J^{131}$ oder $J^{131}$, welches in beliebiger Kombination in der Gruppe W oder G oder V vorliegt, werden am besten für eine Radioisotopenkontrastierung von bösartigen Neubildungen verwendet.

Besonders bevorzugt ist die Verwendung von Fluoronderivaten der allgemeinen Formel I, worin

$R_1$ und $R_8$ beide J oder einer J und der andere Wasserstoff (H), F, Cl, Br, $CH_3$, $CF_3$, $CCl_3$, $C_2H_5$, COOH, $COOCH_3$, $NO_2$, $NH_2$, OH, $OCH_3$ oder $OC_2H_5$ bedeuten;

$R_2$, $R_4$, $R_5$ und $R_6$ alle J oder einer bis drei J und der Rest Wasserstoff (H), F, Cl, Br, A, B, W, G, AW, AG, BW, BG, $CH_3$, $CF_3$, $CCl_3$, $CBr_3$, COOH, COOA,COOB, OA, OB, $NO_2$, $NH_2$, COOH, COOA, COOB, $NO_2$ und $NH_2$ bedeuten;

$R_3$ Wasserstoff (H), Kation eines Alkalimetalls oder Ammonium, A, B, W, G, Q, Z oder Glukoronsäure bedeutet;

$R_8$ Wasserstoff (H), oder J oder J, welches in beliebiger Kombination in Gruppen A, B, W, G, AW, AG, BW, BG, I, K, AI, AK, BI, BK, WI, GI, GK, L, M , LM, Y, V, AY, AV, BY, BV, WY, WV, GY und GV ist, für eine Radioisotopenkontrastierung von bösartigen Neubildungen.

Weiterhin ist die Verwendung von Fluoronderivaten der allgemeinen Formel I, worin $R_1$ und $R_7$ aus der Gruppe Wasserstoff (H),$CH_3$, $NO_2$, $NH_2$, OH und $OCH_3$ ausgewählt sind;

$R_2$, $R_4$, $R_5$ und $R_6$ alle J in beliebiger Kombination mit Wasserstoff (H), F, Cl, $CH_3$, $CF_3$, $CCl_3$, $CJ_3$, $NO_2$ oder $NH_2$ bedeuten;

$R_3$ Wasserstoff (H), Kation eines Alkalimetalls oder Ammonium bedeutet;

$R_8$ Wasserstoff (H) oder J oder J, welches in beliebiger Kombination in die Gruppen W oder G oder V stellt, charakteristisch für eine Röntgenkontrastierung von bösartigen Neubildungen.

Fluoronderivate der allgemeinen Formel I, worin

$R_1$ und $R_7$ F oder einer F und der andere Wasserstoff (H), Cl, Br, J , $CH_3$, $CF_3$, $CCl_3$, $C_2H_5$, COOH, $COOCH_3$, $NO_2$,$NH_2$, OH, $OCH_3$ oder $OC_2H_5$ bedeuten;

$R_2$, $R_4$, $R_5$ und $R_6$ alle F oder eins bis drei F und der Rest eins oder mehrere aus der Gruppe Wasserstoff (H), Cl, Br, J, A, B, W, G, AW, AG, BW, BG, $CH_3$, $CF_3$, $CCl_3$, $CBr_3$, $CJ_3$, COOH, COOA, COOB, OA, OB, $NO_2$ und $NH_2$ bedeuten,

$R_3$ Wasserstoff (H), das Kation eines Alkalimetalls oder Ammonium, A, B, W, G, Q, Z oder Glukoronsäure bedeutet;

$R_8$ Wasserstoff (H) oder F oder F, welches in beliebiger Kombination in den Gruppen A, B, W, G, AW, AG, BW, BG, I, K, AI, BI, BK, AK, WI, WK, GI, GK, L, M, LM, Y, V, AY, BY, BV, AV, WY, GY, WV und GV vorliegt, werden für eine NMR-Kontrastierung von bösartigen Neubildungen ebenfalls bevorzugt.

Bevorzugt ist auch die Verwendung von Fluoronderivaten der allgemeinen Formel I, worin

$R_1$ und $R_7$ F oder einer F und der andere Wasserstoff (H), Cl, $CH_3$, $CF_3$, $NO_2$ oder $NH_2$ bedeuten;

$R_2$, $R_4$, $R_5$ und $R_6$ alle F oder eins bis 3 F und der Rest eins oder mehrere aus der Gruppe Wasserstoff (H), $CF_3$, $NO_2$, $NH_2$ und OH bedeuten;

$R_3$ Wasserstoff (H), Kation eines Alkalimetalls oder Ammonium bedeutet;

$R_8$ F oder die Gruppe A, substituiert durch F von eins bis zur vollständigen Substitution, die Gruppe B, substituiert durch F von eins bis zur vollständigen Substitution, die Gruppe I, substituiert durch F von eins bis zur vollständigen Substitution, die Gruppe Y, substituiert durch F, $CF_3$ von eins bis zur vollständigen Substitution, bei beliebiger Kombination unter ihnen, für eine NMR-Kontrastierung von bösartigen Neubildungen.

Fluoronderivate der allgemeinen Formel I, worin

$R_1$ und $R_7$ jeder mindestens eines aus der Gruppe Wasserstoff (H), F, Cl, $CH_3$,$CF_3$, $CCl_3$, COOH, $COOCH_3$, $NO_2$, $NH_2$, OH, $OCH_3$ oder $OC_2H_5$ bedeuten;

$R_2$, $R_4$, $R_5$ und $R_6$ aus der Gruppe Wasserstoff (H), F, Cl, die Gruppe A, die Gruppe A, substituiert durch F, Cl, von eins bis zur vollständigen Substitution bei beliebiger Kombination unter ihnen, $CH_3$, $CF_3$, $CCl_3$, COOH, COOA, COOB, OA, OB, $NO_2$, $NH_2$ und W bedeuten;

Gruppe X - die Gruppe W, substituiert von eins bis zur vollständigen Substitution durch F, Cl, $NO_2$, $NH_2$, OH, $HSO_3^-$ , COOH oder $COO^-$ bedeutet;

$R_3$ Wasserstoff (H), Kation eines Alkalimetalls oder Ammonium, A, P, W, X, Q oder Acyl (R-CH=O) bedeutet;

$R_8$ Wasserstoff (H), A, P, W, X, AW. AX, PW, PX und I,

Gruppe T die Gruppe I, substituiert durch F, Cl, $NO_2$, $NH_2$, OH, $HSO_3^-$ , COOH, $COO^-$. von eins bis zur

vollständigen Substituierung bei beliebiger Kombination unter ihnen, AI, PI, AT, PT, WI, XI, WT, XT, L, M, LM, Y, Gruppe U die Gruppe Y, substituiert durch $NO_2$, $NH_2$, OH, COOH, $COO^-$, $HSO_3^-$, F, Cl, COOA, COOX,

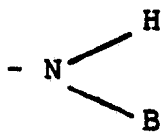 , 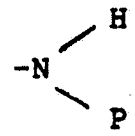 ,

$CH_3$, $CF_3$, $CCl_3$ von eins bis zur vollständigen Substitution in beliebiger Kombination unter ihnen, AY, PY, AU, PU, WY, XU, XY oder WU ist, sind vorzüglich für eine Fluoreszenzkontrastierung von bösartigen Neubildungen geeignet.

Die Anwendung von Fluoronderivaten der allgemeinen Formel I, worin $R_1$ und $R_7$ aus der Gruppe Wasserstoff (H), F, Cl, $NO_2$, $NH_2$ und OH ausgewählt sind;
$R_2$, $R_4$, $R_5$ und $R_6$ aus der Gruppe Wasserstoff (H), F, Cl, $NO_2$, $NH_2$ und OH bedeuten;
$R_3$ Wasserstoff (H), das Kation eines Alkalimetalls oder Ammonium bedeutet;
$R_8$ Wasserstoff (H), die Gruppe W, X, I, T, Y oder U ist, ist für eine Fluoreszenzkontrastierung von bösartigen Neubildungen besonders bevorzugt.

Die Verbindungen der oben angeführten Formel weisen eine Fähigkeit auf, sich selektiv in großen Konzentrationen in lebendigen bösartigen Geschwulsten statt in normalen Zellen und Geweben zu akkumulieren.

Es ist zweckmäßig, die Fluoronderivate in einer Menge von 0,1 bis 20 mg je 1 kg der Masse eines lebendigen Organismus anzuwenden. Die untere Grenze der Konzentrationen wird durch die Empfindlichkeit der Methode zur Registrierung der Fluoronderivate in Geweben und Zellen bestimmt. Die obere Grenze der Konzentrationen ist mit der begrenzten Lösbarkeit der Fluoronderivate verbunden. Die Ergebnisse der Versuche an Labortieren haben gezeigt, daß im Bereich der Konzentrationen von 0,1 bis 20,0 mg/kg der Masse die Fluoronderivate keine toxische Wirkung ($LD_{50} > 300$ mg/kg der Masse) hervorrufen.

Ein höherer Gehalt im erfindungsgemäßen Mittel an Kohlenhydraten und/oder Vitaminen und/oder Blockatoren der Durch dringungsfähigkeit der Zellmembranen führt zu einer beträchtlichen Erhöhung des Akkumulierungskontrastes der Fluoronderivate in bösartigen Zellen und Geschwulsten im Vergleich zu normalen Zellen und Geweben.

Der Wirkungsmechanismus dieser Zusätze, der im voraus durchaus nicht offensichtlich war, besteht im folgenden. Die bösartigen Zellen sind durch einen glykolytischen Typ der Atmung gekennzeichnet, für welchen ein erhöhter Verbrauch von Kohlenhydraten und eine Einsäuerung der Zwischengewebeflüssigkeit durch einen Auswurf organischer Säuren als Endprodukte der Atmung charakteristisch sind. Es wurde festgestellt, daß das Vorhandensein von Glykolyseprodukten im die Zelle umfassenden Medium sowie die Entsäuerung des Mediums zu einer Erhöhung der Lösbarkeit der Fluoronderivate in Zellmembranen und folglich zu einer starken Steigerung seiner Konzentration im Inneren der bösartigen Zellen führt.

Als Kohlenhydrate sind Glukose oder Fruktose (Monosaccharide) sowie Saccharose (Disaccharid) bevorzugt und die Wahl wird durch konkrete physiologische Besonderheiten des Organismus, z.B. wenn der Patient an Diabetes leidet, wird im Mittel Fruktose, aber nicht Glukose empfohlen, bedingt.

Der Zusatz von Vitaminen, und zwar des A-Retinols, des $B_1$ Thiamins, des $B_2$-Riboflavins, des $B_6$-Pyridoxin-Chlorids, des P-Rutins, E-Tokopherol-Acetats, der C-Ascorbinsäure, der PP-Nikotinsäure führt zu einer gemeinsamen Stimulierung von Stoffwechselprozessen, insbesondere der Glykolyse der bösartigen Zellen und der Erhöhung der Durchdringungsfähigkeit der Zellmembranen, die für einen aktiven. Transport der Fluoronderivate in die bösartigen Zellen notwendig sind.

Der Bereich der Vitaminkonzentrationen entspricht den üblichen Tagesdosen. Die Ausschließung einiger Vitamine aus der Mischung kann mit individuellen Besonderheiten des Organismus verbunden sein. Dabei kann der Kontrast der Akkumulierung eines Fluoronderivats in bösartigen Geweben gesenkt werden.

Die Anwendung der Blockatoren der Durchdringungsfähigkeit von Zellmembranen, vorzugsweise von Antihistaminpräparaten, beispielsweise von 3-Methyl-9-benzyl-1,2,3,4-tetrahydrocarbolin-naphthalin-1,5-disulfanat oder Hydrochlorid des 10-(2-Dimethylaminopropyl)-phenothiazins oder Hydrochlorid des N-Dimethylaminoethyl-N-(para-chlorbenzyl)aminopyridins oder 1-Methyl-2-[2-(α-methyl-para-chlorbenzhydryloxy)-ethyl]pyrromedin oder 4,9-Dihydro-4-(1-methyl-4- piperidinyliden)I-OH-benzo-[4-,5]-cyclohepta[1,2]-thiophen-10-OH(hydrofumarat) oder Hydrochlorid des ß-Dimethylaminoethylethers des Benzhydrols oder Hydrochlorids des Chinuklydyl-3(diphenyl)carbinols ist auf die Verringerung der Geschwindigkeit des Austritts der Fluoronderivate aus den bösartigen Zellen gerichtet. Dabei wird auch die Erhöhung der

Konzentration der Fluoronderivate in den bösartigen Zellen gewährleistet und als Folge der positive Effekt, und zwar ein erhöhter Kontrast der Akkumulierung der Fluoronderivate in einer bösartigen Geschwulst erreicht. Somit gewährleisten die anzuwendenden Zusatzstoffe sowohl die Erhöhung einer selektiven Akkumulierung der Fluoronderivate als auch die Senkung der Geschwindigkeit ihres Austritts aus den Zellen. Es sei hervorgehoben, daß sich die Selektivität dieses Prozesses, die durch die Besonderheiten des Metabolismus der bösartigen Zellen bedingt ist, im ganzen in einem großen Kontrast der Akkumulierung der Fluoronderivate in bösartigen Geweben gegenüber den normalen gezeigt hat.

Unter Berücksichtigung individueller Besonderheiten des Organismus ist es möglich, folgende Varianten der Zusammensetzungen des Mittels zur Kontrastierung (in Gew.-%) anzuwenden:

1. Volle Zusammensetzung des Mittels

| | |
|---|---|
| Fluoronderivate | 0,1 bis 89 |
| Saccharide und/oder Disaccharide | 10,0 bis 98,9 |
| Vitamine | 0,5 bis 89,4 |
| Blockatoren | 0,5 bis 89,4 . |

Die bevorzugte volle Zusammensetzung des Mittels ist die folgende (in Gew.-%):

| | |
|---|---|
| Fluoronderivate | 5 |
| Saccharide und/oder Disaccharide | 90 |
| Vitamine | 2,5 |
| Blockatoren | 2,5. |

2. Zusammensetzung des Mittels ohne Saccharide und/oder Disaccharide

| | |
|---|---|
| Fluoronderivate | 1 bis 90 |
| Vitamine | 6 bis 96 |
| Blockatoren | 3 bis 93. |

Die bevorzugte Zusammensetzung des Mittels ohne Saccharide und/oder Disaccharide ist die folgende (in Gew.-%):

| | |
|---|---|
| Fluoronderivate | 88 |
| Vitamine | 10 |
| Blockatoren | 2. |

3. Zusammensetzung des Mittels ohne Vitamine

| | |
|---|---|
| Fluoronderivate | 0,1 bis 89 |
| Saccharide und/oder Disaccharide | 10 bis 98,9 |
| Blockatoren | 1,0 bis 88,9. |

Die bevorzugte Zusammensetzung des Mittels ohne Vitamine stellt die folgende dar (in Gew.-%):

| | |
|---|---|
| Fluoronderivate | 6 |
| Saccharide und/oder Disaccharide | 92 |
| Blockatoren | 2. |

4. Zusammensetzung des Mittels ohne Blockatoren

| | |
|---|---|
| Fluoronderivate | 0,1 bis 89 |
| Saccharide und/oder Disaccharide | 10 bis 98,9 |
| Vitamine | 1,0 bis 88,9. |

Die bevorzugte Zusammensetzung des Mittels ohne Blockatoren stellt die folgende dar (in Gew.-%):

| | |
|---|---|
| Fluoronderivate | 8 |
| Saccharide und/oder Disaccharide | 90 |
| Vitamine | 2. |

5. Zusammensetzung des Mittels ohne Vitamine und Blockatoren

| | |
|---|---|
| Fluoronderivate | 0,1 bis 80 |
| Saccharide und/oder Disaccharide | 20 bis 99,9. |

Die beste Zusammensetzung des Mittels ohne Vitamine und Blockatoren ist die folgende (in Gew.-%):

| | |
|---|---|
| Fluoronderivate | 10 |
| Saccharide und/oder Disaccharide | 90. |

6. Zusammensetzung des Mittels ohne Saccharide und/oder Disaccharide und Vitamine

| | |
|---|---|
| Fluoronderivate | 20 bis 90 |
| Blockatoren | 10 bis 80. |

Die bevorzugte Zusammensetzung des Mittels ohne Saccharide und/oder Disaccharide und Vitamine ist die folgende (in Gew.-%):

| | |
|---|---|
| Fluoronderivate | 82 |
| Blockatoren | 18. |

7. Zusammensetzung des Mittels ohne Saccharide und/oder Disaccharide und Blockatoren

| | |
|---|---|
| Fluoronderivate | 1 bis 90 |
| Vitamine | 10 bis 99. |

Die bevorzugte Zusammensetzung des Mittels ohne Saccharide und/oder Disaccharide und Blockatoren ist die folgende (in Gew.-%):

| | |
|---|---|
| Fluoronderivate | 82 |
| Vitamine | 18. |

8. Zusammensetzung des Mittels ohen Saccharide und/oder Disaccharide und Vitamine

| | |
|---|---|
| Fluoronderivate | 100. |

Aus allen möglichen Kombinationen ist die volle Zusammensetzung des Mittels am besten. Die restlichen Zusammensetzungen werden durch den physiologischen Zustand des Patienten bestimmt, z.B. durch eine mögliche Allergie gegen irgendwelche Komponenten, durch Krankheiten (Diabetes) oder eine Dysfunktion der inneren Organe.

Wird eine der Komponenten der erfindungsgemäßen Mittels entsprechend den oben angeführten Zusammensetzungen, selbstverständlich außer dem Fluoronderivat ausgeschlossen, bleibt die Selektivität seiner Akkumulierung in bösartigen Geschwulsten erhalten, aber auf einem niedrigeren Niveau (s. Tabelle 1).

Man kann das Mittel zur Kontrastierung von bösartigen Neubildungen durch intravenöse Injektionen, enteral oder über den Anus einführen. Es gibt auch andere Varianten: man kann z.B. die Vitamine und die Kohlenhydrate enteral einnehmen und die Fluoronderivate intravenös einführen.

Einzelne Komponenten des erfindungsgemäßen Mittels zur Kontrastierung können entweder in Form einer Lösung aller Komponenten oder eines Pulvers oder in Form von individuellen Lösungen oder in fester Form angewendet werden (auch in beliebiger Reihenfolge).

Als Lösungsmittel für das Kontrastmittel im ganzen oder für seine einzelnen Komponenten kann Wasser oder eine 5 bis 30%ige wässerige ethanolische Lösung angewendet werden.

In Abhängigkeit von der Lösbarkeit der Fluoronderivate können auch Öllösungen - Solutio oleosa - und alkoholische Zuckerlösungen - Solutio spirituosa-saccharum - angewendet werden. Die öligen Lösungen werden auf der Grundlage von Rizinus-, Oliven-, Sonnenblumen-, Baumwollsamen-, Pfirsichöl und der anderen Öle, die für eine enterale Einführung in der Pharmakologie angewendet werden, hergestellt.

Das erfindungsgemäße Mittel gewährleistet eine hohe Kontrastierung von bösartigen Geschwulsten durch seine selektive Akkumulierung in erhöhten Konzentrationen in diesen Geschwulsten gegenüber den normalen Geweben. Der Einsatz der Fluoronderivate mit einem hohen Austritt der Fluoreszenz ist optimal für eine Fluoreszenzkontrastierung von bösartigen Geschwulsten. Die Anwendung der Fluorderivate von Fluoron gewährleistet eine Kontrastierung bösartiger Geschwulste bei NMR-Tomografieuntersuchungen; dabei werden Geschwulste beliebiger Lokalisation entdeckt. Die Jod-Derivate des Fluorons gewährleisten eine Kontrastierung bösartiger Neubildungen bei Röntgenuntersuchungen, einschließlich der Röntgen-Tomografie. Die Anwendung von Derivaten des Isotops $J^{131}$ erfolgt bei der radioisotopen Diagnostik der Geschwulste.

Die Fluoronderivate werden in an sich bekannter Weise erhalten, z.B. durch eine ein- oder zweistufige Kondensation von Phthalsäureanhydrid mit Resorzinderivaten oder durch Kondensation von verschiedenen Resorzinderivaten und Aldehyden sowie durch eine Behandlung der Xanthonfarbstoffe mit entsprechenden Reaktanten.

Die Bestimmung der Fähigkeit der Fluoronderivate, sich in bösartigen Zellen und Geschwulsten zu akkumulieren, wurde auf Zellkulturen der He-, Za-, ZTZ-, Z-Fibroplaste, welche nach Standardmethoden kultiviert werden, durchgeführt. Nachdem der Zellkultur ein Fluoronderivat neben den anderen Komponenten des Mittels zugesetzt und innerhalb eines bestimmten Zeitabschnitts inkubiert worden war wurde eine Zellenfraktion entnommen und nach dem Waschen in den Zellen die Konzentration des Fluoronderivats unter Anwendung der Methoden einer chromatografischen und einer Fluoreszenzanalyse bestimmt.

In Versuchen mit Labortieren wurden Mäuse CBA, C-5EB, Black und BALB-C mit umgeimpften Geschwulsten carciona cervix, Lewis tumor, carciona intestinum crassum getestet. Nach einer bestimmten Zeit nach der Einführung der Fluoronderivate neben den anderen Komponenten des Mittels wurden die Tiere unter Narkose getötet und die Konzentration des Fluoronderivats in der Geschwulst und in normalen Geweben bestimmt. Der Kontrast der Akkumulierung der Fluoronderivate wurde als Verhältnis der Konzentrationen des Präparats im bösartig/normalen Gewebe bestimmt.

Bei der Anwendung von Jod- und Fluorderivaten des Fluorons wurden die Methoden der Röntgen- und NMR-Tomografie für die Bewertung der kontrastierenden Fähigkeit der Präparate angewendet.

Bei laparoskopischen Untersuchungen wurde festgestellt, daß sich die Fluoronderivate in der Aszitflüssigkeit akkumulieren, was es gestattet, diese in sehr geringen Mengen zu entdecken und zu dem Schluß zu kommen, daß es in Organen bösartige Tiefmetastasen, die nicht visuell entdeckt werden können, z.B. in der Leber, im Bauchgebiet, in der Milz u.a. gibt, was diagnostische Möglichkeiten der Laparoskopie wesentlich erweitert.

Die im Blut anwesenden bösartigen Elemente akkumulieren die Fluoronderivate und können nach der Kontrastierung, z.B. einer Fluoreszenzkontrastierung unter dem Mikroskop entdeckt werden.

Man beginnt die Untersuchung der Lokalisation der Fluoronderivate im Gewebe des Organismus nach einer bestimmten Zeit nach der Einführung des Präparats, da sich die Fluoronderivate innerhalb dieser Zeit zuerst in allen Geweben gleichmäßig verbreiten und erst danach in den bösartigen Geschwulsten akkumulieren. Das Austreten aus den normalen Geweben über die Leber und die Nieren beginnt praktisch sofort nach der Einführung des Präparats. Nach einer bestimmten Zeit, die für die Lokalisation, den Typ und die Größe der bösartigen Geschwulst charakteristisch ist, wird in der letzteren eine erhöhte Konzentration des Präparats festgestellt. Die Feststellung der Lokalisation der Fluoronderivate kann durch Fluoreszenz- und Radioisotopanalyse, auf röntgenologischem Wege oder durch die NMR- oder Röntgentomografie erfolgen.

Die Gewebe mit einer erhöhten Konzentration des Fluorons wurden einer histologischen Analyse zwecks Belegung der bösartigen Natur des Gewebes unterworfen. Es wurde festgestellt, daß die Richtigkeit der Diagnostik eines bösartigen Vorgangs nach der Bestimmung der Fluoronderivate in den Geweben im Vergleich zu einer üblichen histologischen Analyse eines Biopsiematerials 75 bis 95 % in Abhängigkeit von der Lokalisation der Geschwulst beträgt.

Um das Wesen der Erfindung besser zu verstehen, sind in der Tabelle 1 Beispiele des Mittels zur Kontrastierung von bösartigen Neubildungen angeführt.

Tabelle 1

Substituenten $R_1$ + $R_8$ in der allgemeinen Formel der Fluoronderivate

| Nr. der Verbindung | $R_1$ | $R_2$ | $R_3$ | $R_4$ | $R_5$ | $R_6$ | $R_7$ | $R_8$ |
|---|---|---|---|---|---|---|---|---|
| 1 | 2 | 3 | 4 | 5 | 6 | 7 | 8 | 9 |
| 1 | H | H | H | H | H | $COOC_2H_5$ | H | 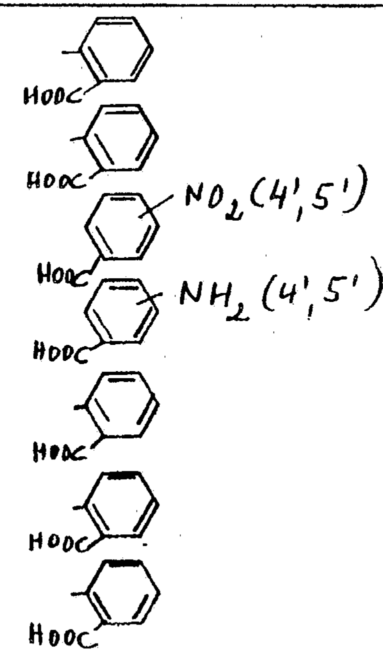 |
| 2 | H | $-CH_3$ | H | H | H | $COOCH_3$ | H | |
| 3 | H | H | H | H | H | H | H | |
| 4 | H | H | H | H | H | H | H | |
| 5 | H | H | H | Br | Br | OH | H | |
| 6 | H | Br | H | COOH | H | Br | H | |
| 7 | H | $HOOCCH_2$<br>$N-CH_2-H$<br>$HOOCCH_2$ | H | $HOOCCH_2$<br>$N-CH_2-H$<br>$HOOCCH_2$ | H | H | H | |

Tabelle 1 (Fortsetzung)

| Nr. der Verbindung | $R_1$ | $R_2$ | $R_3$ | $R_4$ | $R_5$ | $R_6$ | $R_7$ | $R_8$ |
|---|---|---|---|---|---|---|---|---|
| 1 | 2 | 3 | 4 | 5 | 6 | 7 | 8 | 9 |
| 8 | H | $(HOOCCH_2)(HOOCCH_2)N-CH_2-H$ | H | H | H | $(NaOOCCH_2)(HOOCCH_2)N-CH_2-$ | H | 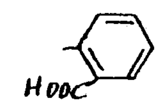 |
| 9 | H | Br | H | Br | Br | Br | H | 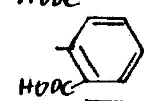 |
| 10 | H | Br | H | Br | Br | Br | H | 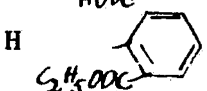 |
| 11 | H | $NO_2$ | H | Br | Br | $NO_2$ | H | 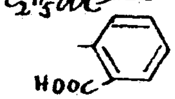 |
| 12 | H | Br | H | Br | Br | Br | H | 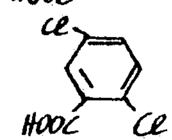 |

Tabelle 1 (Fortsetzung) Zusammensetzung des erfindungsgemäßen Mittels, in Gew.-%

| Nr. der Verbindung | Fluoronderivat | Glykose | Fruktose | Saccharose | Vitamine (s. Anlage zur Tabelle) | | | | Blockator der Durchdringungsfähigkeit der Zellmembran 3-Methyl-9-benzyl-1,2,3,4-tetrahydrocarbonyl-naphthalin-1,5-disulfonat-18 | Kontrast der Akkumulierung des Fluoronderivats in der bösartigen Geschwulst im Vergleich zum normalen Gewebe in relativen Einheiten |
|---|---|---|---|---|---|---|---|---|---|---|
| | | | | | Zusammensetzung I | Zusammensetzung II | Zusammensetzung III | Zusammensetzung IV | | |
| 1 | 10 | 11 | 12 | 13 | 14 | 15 | 16 | 17 | 18 | 19 |
| 1 | 0,1 | 98,9 | - | - | 0,5 | - | - | - | 0,5 | 6,0 |
| 2 | 89,0 | - | 10,0 | - | - | 0,5 | - | - | 0,5 | 4,0 |
| 3 | 0,1 | - | - | 10,0 | - | - | 89,4 | - | 89,4 | 4,0 |
| 4 | 10,0 | 70,0 | - | - | 10,0 | - | - | - | 10,0 | 8,0 |
| 5 | 5,0 | - | 40,0 | 30,0 | 15,0 | - | - | - | 10,0 | 7,0 |
| 6 | 100,0 | - | - | - | - | - | - | - | - | 2,0 |
| 7 | 50,0 | 20,0 | - | - | - | - | - | 20,0 | 10,0 | 3,0 |

Tabelle 1 (Fortsetzung)

| 1 | 10 | 11 | 12 | 13 | 14 | 15 | 16 | 17 | 18 | 19 |
|---|---|---|---|---|---|---|---|---|---|---|
| 8 | 1,0 | 80,0 | – | 10,0 | 5,0 | – | – | – | 4,0 | 8,0 |
| 9 | 1,0 | – | – | – | 6,0 | – | – | – | 93,0 | 3,0 |
| 10 | 15,0 | – | – | – | – | 30,0 | – | – | 55,0 | 4,5 |
| 11 | 1,0 | – | – | – | – | – | 96,0 | – | 3,0 | 2,5 |
| 12 | 0,1 | 10,0 | – | – | – | – | – | – | 88,9 | 5,0 |

| I | I2 | I3 | I4 | I5 | IC | I7 | I8 | 9 | I10 | I11 | I12 | I13 | I14 | I15 | I16 | I17 | I18 | I19 |
|---|---|---|---|---|---|---|---|---|---|---|---|---|---|---|---|---|---|---|
| I3 | H | H | H | H | H | H | H | 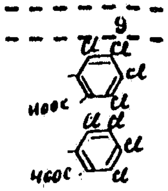 | 0,I | 30,0 | - | - | - | - | - | - | I9,9 | 6,5 |
| I4 | H | Br | H | Br | Br | Br | H | | 0,I | - | 70,0 | - | - | - | - | - | 20,9 | 6,0 |
| I5 | H | H | H | J | J | H | H | 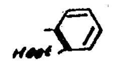 | 50,0 | - | - | 40,0 | - | - | - | - | I0,0 | 6,0 |
| I6 | H | J | H | J | J | J | H | 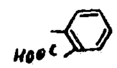 | 0,I | - | 93,9 | - | - | - | - | - | I,0 | 6,5 |
| I7 | H | J | H | J | J | J | H | 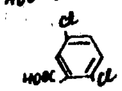 | I,0 | 30,0 | 50,0 | - | - | - | - | - | I9,0 | 5,0 |
| I8 | H | J | H | J | J | J | H | 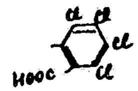 | 5,0 | - | 40,0 | 20,0 | - | - | - | - | 35,0 | 4,5 |
| I9 | H | H | H | OH | OH | H | H | 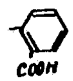 | I,0 | I0,0 | - | - | - | 89,0 | - | - | - | 8,0 |
| 20 | H | H | H | H | H-CH2-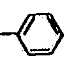 | H | H | -"- | 0,5 | - | 80,0 | - | I9,5 | - | - | - | - | 9,5 |
| 21 | H | Br | H | Br | Br | Br | H | 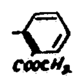 | 0,I | - | - | 93,9 | - | - | - | I,0 | - | 3,5 |
| 22 | OH | OH | H | H | OH | OH | H | 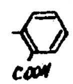 | 0,I | I0,0 | - | - | - | - | 83,9 | - | - | 5,5 |
| 23 | H | OH | H | H | H | OH | H | $-CH_3$ | I0,0 | - | 30,0 | - | 60,0 | - | - | - | - | 3,5 |
| 24 | H | OH | H | H | H | OH | H | $-CCl_3$ | 20,0 | 75,0 | - | - | - | 5,0 | - | - | - | 4,0 |
| 25 | H | OH | H | H | H | OH | H | $-C_2H_5$ | 0,I | 20,0 | 20,0 | 20,0 | - | - | - | 39,9 | - | 3,5 |
| 26 | H | OH | H | H | H | OH | H | $-CH_2-CH_2-CH_3$ | 0,5 | - | 70,0 | I0,0 | - | - | I9,5 | - | - | 3,0 |

| I | I2 | I3 | I4 | I5 | I6 | I7 | I8 | 9 | I10 | I11 | I12 | I13 | I14 | I15 | I16 | I17 | I18 | I19 |
|---|---|---|---|---|---|---|---|---|---|---|---|---|---|---|---|---|---|---|
| 27 | H | OH | H | H | H | OH | H | $-CH_2-C(CH_3)-CH_2-CH_2-CH-C(CH_3)_2$ | 5,0 | 20,0 | - | - | 75,0 | - | - | - | - | 4,0 |
| 28 | H | OH | H | H | H | OH | H/ | $-C_{15}H_{31}$ | 1,0 | 80,0 | - | - | - | 19,0 | - | - | - | 2,0 |
| 29 | H | OH | H | H | H | OH | H | 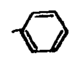 | 0,1 | 99,9 | - | - | - | - | - | - | - | 6,0 |
| 30 | H | OH | H | H | H | OH | H | 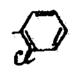 | 0,5 | - | 99,5 | - | - | - | - | - | - | 6,5 |
| 31 | H | OH | H | H | H | OH | H | 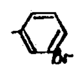 | 10,0 | - | - | 90,0 | - | - | - | - | - | 5,5 |
| 32 | H | OH | H | H | H | OH | H | 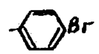 | 20,0 | 80,0 | - | - | - | - | - | + | - | 5,0 |
| 33 | H | OH | H | H | H | OH | H | 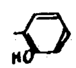 | 80,0 | - | 20,0 | - | - | - | - | - | - | 7,5 |
| 34 | H | OH | H | H | H | OH | H | 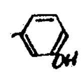 | 1,0 | 60,0 | 39,0 | - | - | - | - | - | - | 8,0 |
| 35 | H | OH | H | H | H | OH | H | 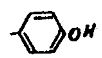 | 5,0 | 95,0 | - | - | - | - | - | - | - | 10,0 |
| 36 | H | OH | H | H | H | OH | H | 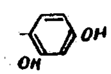 | 1,0 | 15,0 | 50,0 | 34,0 | - | - | - | - | - | 14,0 |
| 37 | H | OH | H | H | H | OH | H | 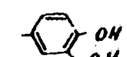 | 1,0 | 80,0 | 9,0 | - | - | - | - | - | - | 10,0 |
| 38 | H | OH | H | H | H | OH | H | 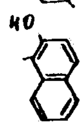 | 5,0 | - | - | 95,0 | - | - | - | - | - | 4,0 |
| 39 | H | OH | H | H | H | OH | H | 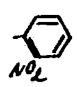 | 1,0 | - | - | - | - | 99,0 | - | - | - | 4,0 |

| I | I2 | I3 | I4 | I5 | I6 | I7 | I8 | 9 | I10 | I11 | I12 | I13 | I14 | I15 | I16 | I17 | I18 | I19 |
|---|---|---|---|---|---|---|---|---|---|---|---|---|---|---|---|---|---|---|
| 40 | H | OH | H | H | H | OH | H | 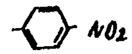 | 1,0 | – | – | – | – | – | – | 90,0 | – | 3,5 |
| 41 | H | OH | H | H | H | OH | H | 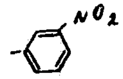 | 1,0 | – | – | – | – | – | 99,0 | – | – | 4,5 |
| 42 | H | OH | H | H | H | OH | H | 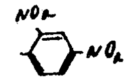 | 1,0 | – | – | – | 99,0 | – | – | – | – | 5,0 |
| 43 | H | OH | H | H | H | OH | H | 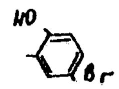 | 90,0 | – | – | – | 10,0 | – | – | – | – | 3,5 |
| 44 | H | OH | H | H | H | OH | H | 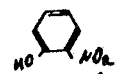 | 90,0 | – | – | – | – | 10,0 | – | – | – | 4,5 |
| 45 | H | OH | H | H | H | OH | H | 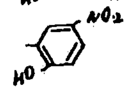 | 90,0 | – | – | – | – | – | – | 10,0 | – | 4,5 |
| 46 | H | OH | H | H | H | OH | H | 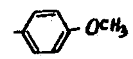 | 5,0 | – | – | – | – | 95,0 | – | – | – | 3,5 |
| 47 | H | OH | H | H | H | OH | H | 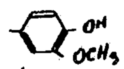 | 5,0 | – | – | – | 95,0 | – | – | – | – | 4,5 |
| 48 | H | OH | H | H | H | OH | H | 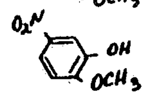 | 10,0 | – | – | – | – | – | – | 90,0 | – | 4,5 |
| 49 | H | OH | H | H | H | OH | H | 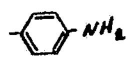 | 20,0 | – | – | – | – | – | – | – | 80,0 | 3,0 |
| 50 | H | OH | H | H | H | OH | H | 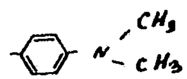 | 80,0 | – | – | – | – | – | – | – | 20,0 | 3,5 |
| 51 | H | OH | H | H | H | OH | H | 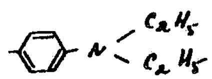 | 90,0 | – | – | – | – | – | – | – | 10,0 | 2,5 |
| 52 | H | OH | H | H | H | OH | H | 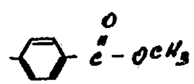 | 100 | – | – | – | – | – | – | – | – | 2,0 |

| I | I2 | I3 | I4 | I5 | I6 | I7 | I8 | 9 | I10 | I11 | I12 | I13 | I14 | I15 | I16 | I17 | I18 | I19 |
|---|---|---|---|---|---|---|---|---|---|---|---|---|---|---|---|---|---|---|
| 53 | H | OH | H | H | H | OH | H | 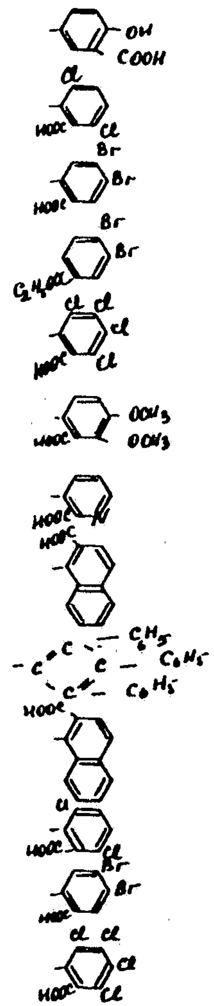 | 0,5 | 80,0 | - | - | - | 5,0 | - | - | I4,5 | 8,0 |
| 54 | H | OH | H | H | H | OH | H | | 0,5 | 20,0 | 60,0 | - | - | - | 10,0 | - | 4,5 | I4, |
| 55 | H | OH | H | H | H | OH | H | | I,0 | 20,0 | - | 60,0 | I0,0 | - | - | - | 9,0 | I5, |
| 56 | H | OH | H | H | H | OH | H | | 5,0 | - | 60,0 | - | - | - | - | I0,0 | 5,0 | 3,0 |
| 57 | H | OH | H | H | H | OH | H | | I,0 | 80,0 | - | - | 5,0 | - | - | - | I4,0 | I3, |
| 58 | H | OH | H | H | H | OH | H | | 5,0 | - | 90,0 | - | - | 2,0 | - | - | 3,0 | 4,0 |
| 59 | H | OH | H | H | H | OH | H | | 0,I | 90,0 | - | - | 5,0 | - | - | - | 4,9 | 3,0 |
| 60 | H | OH | H | H | H | OH | H | | 0,5 | - | 70,0 | - | - | I0,0 | - | - | I9,5 | 5,0 |
| 6I | H | OH | H | H | H | OH | H | | 5,0 | 80,0 | - | - | - | - | I5,0 | - | - | 2,5 |
| 62 | H | OH | H | Br | Br | OH | H | | 0,I | - | - | 80,0 | - | - | - | I6,0 | 4,9 | 4,5 |
| 63 | H | OH | H | Br | Br | OH | H | | I,0 | 75,0 | - | - | 20,0 | - | - | - | 4,0 | I8, |
| 64 | H | OH | H | Br | Br | OH | H | | 5,0 | 75,0 | - | - | I6,0 | - | - | - | 4,0 | I6, |
| 65 | H | OH | H | Br | Br | OH | H | | 0,I | - | 80,0 | - | - | I0,0 | - | - | 9,9 | 20, |

| I | I2 | I3 | I4 | I5 | I6 | I7 | I8 | 9 | I10 | I11 | I12 | I13 | I14 | I15 | I16 | I17 | I18 | I19 |
|---|---|---|---|---|---|---|---|---|---|---|---|---|---|---|---|---|---|---|
| 66 | H | OH | H | H | H | OH | H | 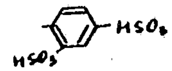 | 5,0 | 90,0 | - | - | - | - | - | - | 5,0 | 6,5 |
| 67 | H | OH | H | H | H | OH | H | 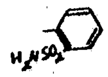 | 10,0 | - | 80,0 | - | - | - | 5,0 | - | 5,0 | 6,5 |
| 68 | H | OH | H | H | H | OH | H | 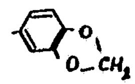 | 0,5 | 10,0 | 80,0 | - | 5,0 | - | - | - | 4,5 | 6,0 |
| 69 | H | H | H | H | H | OH | H | 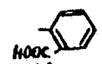 | 1,0 | 90,0 | - | - | - | - | 5,0 | - | 4,0 | 20,0 |
| 70 | H | OH | H | H | H | OH | H | 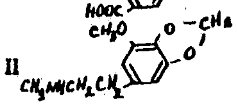 | 1,0 | 25,0 | - | - | 40,0 | - | - | - | 34,0 | 4,0 |
| 71 | H | OH | H | H | H | OH | H | 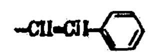 | 1,0 | - | 70,0 | - | - | 20,0 | - | - | 9,0 | 8,0 |
| 72 | H | OH | H | H | H | OH | H | 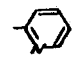 | 10,0 | 80,0 | - | - | - | - | 5,0 | - | 5,0 | 4,5 |
| 73 | H | OH | H | H | H | OH | H | 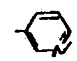 | 10,0 | 80,0 | - | - | - | - | 5,0 | - | 5,0 | 5,0 |
| 74 | H | OH | H | H | H | OH | H | 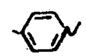 | 10,0 | 80,0 | - | - | - | - | 5,0 | - | 5,0 | 4,6 |
| 75 | H | OH | H | H | H | OH | H | 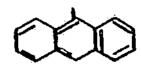 | 10,0 | - | 80,0 | - | - | - | - | 5,0 | 5,0 | 2,5 |
| 76 | H | H | H | H | H | H | $CH_3$ | 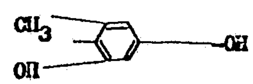 | 1,0 | - | - | 80,0 | 10,0 | - | - | - | 9,0 | 16,0 |
| 77 | H | Br | H | OH | OH | Br | H | 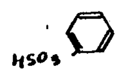 | 5,0 | - | 80,0 | - | - | 6,0 | - | - | 5,0 | 16,0 |
| 78 | H | F | H | F | F | F | H | 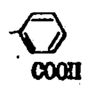 | 1,0 | 80,0 | - | - | - | - | 19,0 | - | - | 20,0 |

| I | I2 | I3 | I4 | I5 | I6 | I7 | I8 | 1 2 | I10 | I11 | I12 | I13 | I14 | I15 | I16 | I17 | I18 | I19 |
|---|---|---|---|---|---|---|---|---|---|---|---|---|---|---|---|---|---|---|
| 79 | H | OH | H | H | H | OH | H | 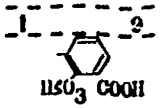 | 1,0 | 80,0 | - | - | - | - | 19,0 | - | - | 15,0 |
| 80 | H | H | $Na^+$ | H | H | F | H | 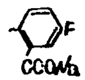 | 0,5 | - | 80,0 | - | - | - | - | 15,0 | 4,5 | 20,0 |
| 81 | H | I | H | $CH_3$ | F | J | H | $CH_3$-$C_{10}H_{21}$ | 0,5 | - | 60,0 | - | - | - | - | 15,0 | 4,5 | 13,5 |
| 82 | $CH_3O$ | H | H | OH | OH | H | H | $-CF_3$ | 0,5 | - | 30,0 | - | - | - | - | 15,0 | 4,5 | 5,0 |
| 83 | $COOCH_3$ | H | H | H | H | H | $NH_2$ | $-CCl_3$ | 0,5 | - | 30,0 | - | - | - | - | 15,0 | 4,5 | 5,5 |
| 84 | OH | F | H | H | H | F | H | $CH_3$ | 10,0 | 70,0 | - | - | 20,0 | - | - | - | - | 5,0 |
| 85 | H | $C_2H_5O$ | H | H | H | H | H | $-CCl_3$ | 5,0 | - | 70,0 | - | - | 20,0 | - | - | 5,0 | 3,5 |
| 86 | Br | H | $CH_3$ | H | H | H | H | 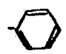 | 5,0 | - | - | 60,0 | - | - | 20,0 | - | 15,0 | 2,5 |
| 87 | H | H | H | $CH_3O$ | H | $C_6H_5$ | H | 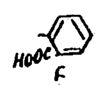 | 1,0 | 80,0 | - | - | - | 5,0 | - | - | 14,0 | 7,5 |
| 88 | $NH_2$ | H | H | H | H | H | $CF_3$ | 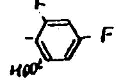 | 5,0 | - | 60,0 | - | 30,0 | - | - | - | 5,0 | 14,0 |
| 89 | H | H | H | OH | H | H | H | 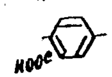 | 1,0 | 90,0 | - | - | - | - | - | 5,0 | 4,0 | 13,0 |
| 90 | H | H | $NH_2$ | H | $NO_2$ | H | H | 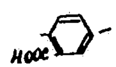 | 1,0 | - | 80,0 | - | - | 19,0 | - | - | - | 12,0 |
| 91 | Cl | H | H | H | $C_6H_5$ | H | H | 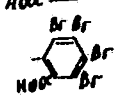 | 10 | 70,0 | - | - | - | - | - | - | 20,0 | 5,0 |

| I | I2 | I3 | I4 | I5 | I6 | I7 | I8 | I | 9 | I10 | I11 | I12 | I13 | I14 | I15 | I16 | I17 | I18 | I19 |
|---|---|---|---|---|---|---|---|---|---|---|---|---|---|---|---|---|---|---|---|
| 92 | H | H | $C_6H_5$ | H | H | COOH | $CH_3$ | | 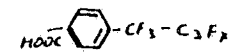 | I,0 | - | - | 80,0 | - | - | - | I0,0 | 9,0 | 3,5 |
| 93 | I | H | H | H | H | H | H | | 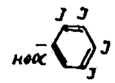 | 3,0 | - | 75,0 | - | - | - | I5,0 | - | 7,0 | I4,0 |
| 94 | H | OH | $C_2H_5$ | $-CH{=}CH-C_6H_5$ | H | $NO_2$ | H | | 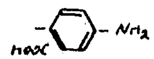 | I,0 | 80,0 | - | - | - | - | - | - | I9,0 | 5,0 |
| 95 | H | H | H | H | H | H | H | | 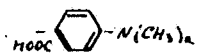 | I,0 | - | 80,0 | - | - | - | I0,0 | - | 9,0 | 6,0 |
| 96 | [illegible] | Br | H | H | H | Br | H | | 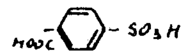 | 5,0 | - | - | 80,0 | - | - | - | 5,0 | I0,0 | 8,0 |
| 97 | $NO_2$ | H | H | H | H | H | H | | 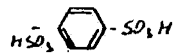 | 5,0 | 70,0 | - | - | I0,0 | - | - | - | I5,0 | 6,0 |
| 98 | H | H | H | H | H | H | H | | 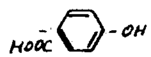 | I,0 | - | 90,0 | - | - | 5,0 | - | - | 4,0 | I8,0 |
| 99 | $CCl_3$ | H | H | H | H | H | H | | 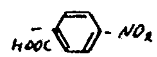 | I,0 | - | 90,0 | - | - | 5,0 | - | - | 4,0 | I6,0 |
| I00 | H | $NH_2$ | H | H | $CF_3$ | $COOC_2H_5$ | H | $-CH{=}CH-$ | 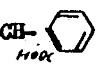 | I,0 | - | - | - | - | - | - | 50,0 | 49,0 | 3,5 |
| I0I | H | H | H | $NH_2$ | H | H | $CH_3$ | | 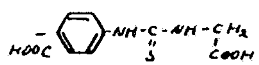 | 5,0 | - | - | - | - | - | 80,0 | - | 65,0 | 2,5 |
| I02 | H | H | $C_3H_7$ | H | H | $CCl_3$ | H | | 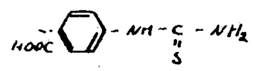 | 5,0 | - | - | - | - | - | 30,0 | - | 65,0 | 3,0 |
| I03 | H | H | H | H | H | H | $CH_3$ | | 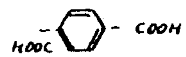 | 5,0 | - | - | - | - | - | 30,0 | - | 65,0 | 8,0 |
| I04 | H | H | H | COOH | H | H | H | | 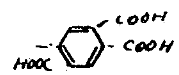 | 5,0 | 80,0 | - | - | I5,0 | - | - | - | - | I2,0 |
| I05 | $CF_3$ | H | $CH_3$ | H | H | H | $NH_2$ | | 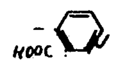 | I,0 | - | 75,0 | - | - | I0,0 | - | - | I4,0 | I0,0 |

| 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|
| 106 | H | Cl | H | H | $CH_3$ | COOH | H | 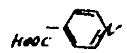 | 100,0 | – | – | – | – | – | – | – | – | 2,0 |
| 107 | H | H | H | $NO_2$ | H | H | H | 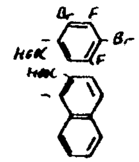 | 1,0 | 80,0 | – | – | 10,0 | – | – | – | 9,0 | 13,0 |
| 108 | H | H | H | H | H | $NH_2$ | H | | 1,0 | – | 60,0 | – | – | – | – | 25,0 | 14,0 | 10,0 |
| 109 | H | H | $CH_3CO$ | H | H | H | H | 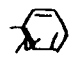 | 5,0 | 10,0 | – | 60 | – | – | 5,0 | – | 10,0 | 5,5 |
| 110 | H | $NO_2$ | H | COOH | H | $NO_2$ | COOH | 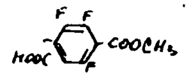 | 1,0 | 60,0 | – | – | 25,0 | – | – | – | 14,0 | 7,0 |
| 111 | H | H | $C_6H_5$ | H | $COOCH_3$ | H | H | 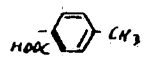 | 5,0 | – | 80,0 | – | – | – | – | 5 | 10,0 | 6,0 |
| 112 | $CBr_3$ | $CH_3$ | $CH_3$ | H | H | $C_6H_5$ | H | 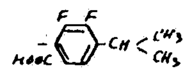 | 0,5 | – | – | 90,0 | – | 5,0 | – | – | 4,5 | 6,0 |
| 113 | H | H | H | H | H | $CBr_3$ | H | 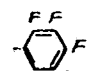 | 1,0 | 80,0 | – | – | – | – | 15,0 | – | 4,0 | 10,0 |
| 114 | H | H | H | H | H | H | H | 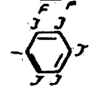 | 1,0 | 80,0 | – | – | – | – | 15,0 | – | 4,0 | 0,0 |
| 115 | H | $CH_3$ | H | H | H | $CH_3$ | H | 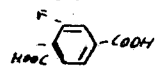 | 5,0 | – | – | 85,0 | – | – | – | – | 10,0 | 6,0 |
| 116 | H | $CH_3$ | $CH_3$ | $CH_3$ | $CH_3$ | $CH_3$ | H | 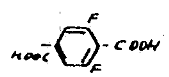 | 5,0 | – | – | 85,0 | – | – | – | – | 10,0 | 3,5 |
| 117 | H | $C_2H_5$ | H | $CH_3$ | $CH_3$ | $C_2H_5$ | H | 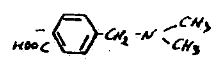 | 1,0 | – | 80,0 | – | 5,0 | – | – | – | 14,0 | 6,0 |
| 118 | $CH_3$ | OH | H | H | H | OH | $CH_3$ | 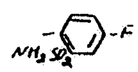 | 40,0 | 50,0 | – | – | – | – | – | – | 10,0 | 8,0 |

| I | I2 | I3 | I4 | I5 | I6 | I7 | I8 | I | 9 | II0 | II1 | II2 | II3 | II4 | II5 | II6 | II7 | II8 | II9 |
|---|---|---|---|---|---|---|---|---|---|---|---|---|---|---|---|---|---|---|---|
| II9 | H | $CH_3$ | H | OH | OH | $CH_3$ | H | | 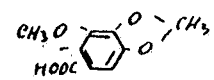 | 20,0 | - | - | 70,0 | - | 10,0 | - | - | - | 3,0 |
| I20 | H | $CF_3$ | H | H | H | $CF_3$ | H | | 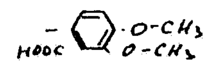 | 1,0 | 85,0 | - | - | - | - | - | 10,0 | 4,0 | 3,0 |
| I21 | H | H | H | $CF_3$ | $CF_3$ | H | H | | 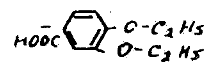 | 1,0 | 85,0 | - | - | - | - | - | 10,0 | 4,0 | 4,5 |
| I22 | H | F | H | $CF_3$ | $CF_3$ | F | H | | 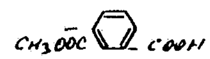 | 10,0 | - | 70,0 | - | - | - | - | 5,0 | 15,0 | 10,0 |
| I23 | F | F | H | $CH_3$ | $CH_3$ | F | F | | 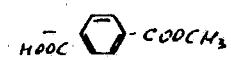 | 10,0 | - | 20,0 | - | 50,0 | - | - | - | 20,0 | 5,0 |
| I24 | H | Br | H | Br | Br | Br | H | | 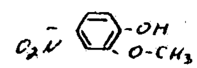 | 1,0 | 80,0 | - | - | - | 19,0 | - | - | - | 7,0 |
| I25 | H | H | H | Br | Br | H | H | | 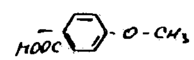 | 1,0 | - | 70,0 | - | - | - | 10,0 | - | 19,0 | 3,0 |
| I26 | H | J | H | J | J | J | H | | 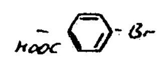 | 0,1 | - | - | 90,0 | - | - | 5,0 | - | 4,5 | 12,0 |
| I27 | $CH_3$ | H | H | J | J | H | H | | 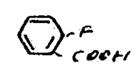 | 0,1 | 90,0 | - | - | - | - | 5,0 | - | 4,9 | 15,0 |
| I28 | H | OH | H | H | H | OH | H | | 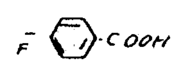 | 0,1 | 90,0 | - | - | - | - | 5,0 | - | 4,9 | 18,0 |
| I29 | H | H | H | OH | OH | H | H | | 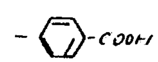 | 0,1 | 90,0 | - | - | - | - | 5,0 | - | 4,9 | 25,0 |
| I30 | $NO_2$ | COOH | H | H | H | COOH | H | | 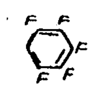 | 1,0 | - | 80,0 | - | - | 5,0 | - | - | 14,0 | 20,0 |
| I31 | H | COOH | H | $CF_3$ | $CF_3$ | COOH | H | | 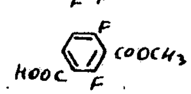 | 1,0 | - | - | 70,0 | - | - | - | 10,0 | 19,0 | 10,0 |

| I | I2 | I3 | I4 | I5 | I6 | I7 | I8 | I | 9 | I10 | I11 | I12 | I13 | I14 | I15 | I16 | I17 | I18 | I19 |
|---|---|---|---|---|---|---|---|---|---|---|---|---|---|---|---|---|---|---|---|
| I32 | H | $COOCH_3$ | H | H | H | $COOCH_3$ | H | 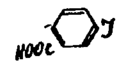 | | 0,1 | 90,0 | - | - | 5,0 | - | - | - | 4,9 | 3,0 |
| I33 | H | H | H | $NH_2$ | $NH_2$ | H | H | 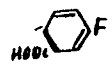 | | 0,5 | - | 90,0 | - | - | - | - | 5,0 | 4,5 | I2,0 |
| I34 | H | H | H | $N(CH_3)_2$ | $N(CH_3)_2$ | H | H | 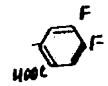 | | 0,5 | - | - | 90,0 | - | - | 5,0 | - | 4,5 | I0,0 |
| I35 | H | $CH_3$ | H | $NH_2$ | $NH_2$ | $CH_3$ | H | 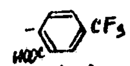 | | 0,I | 80,0 | - | - | I0,0 | - | - | - | 9,9 | 8,0 |
| I36 | $CH_3$ | $CH_3O$ | $CH_3$ | H | H | $CH_3O$ | $CH_3$ | 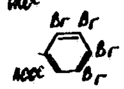 | | I,0 | - | 75,0 | - | - | 5,0 | - | - | I9,0 | I6,0 |
| I37 | $CH_3$ | $CH_3$ | H | $CH_3$ | $CH_3$ | $CH_3$ | $CH_3$ | 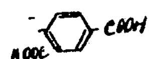 | | I,0 | 95,0 | - | - | - | - | - | - | 4,0 | I4,0 |
| I38 | H | $NO_2$ | H | H | H | $NO_2$ | H | 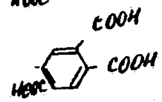 | | I,0 | 95,0 | - | - | - | - | - | - | 4,0 | I5,0 |
| I39 | H | H | H | H | H | COOH | H | 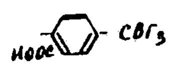 | | 0,I | - | 90,0 | - | 5,0 | - | - | - | 4,9 | I6,0 |
| I40 | F | F | H | F | H | COOH | H | 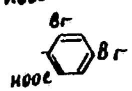 | | I,0 | 90,0 | - | - | - | 4,0 | - | - | 5,0 | I3,0 |
| I41 | H | H | $C_6H_5$ | H | H | H | H | 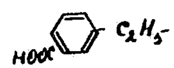 | | 0,I | - | 85,0 | - | - | - | 4,9 | - | I0,0 | 4,5 |
| I42 | H | OH | H | H | H | H | $CH_3$ | 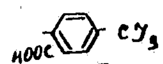 | | 0,5 | - | - | 90,0 | - | - | - | 9,5 | - | 7,0 |
| I43 | H | $CH_3O$ | H | H | H | $CH_3O$ | H | 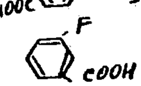 | | 0,5 | 90,0 | - | - | - | - | - | - | 9,5 | I2,0 |
| I44 | H | $CH_3O$ | $CH_3$ | H | H | OH | H | 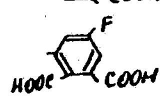 | | 0,5 | - | 90,0 | - | 9,5 | - | - | - | - | 6,0 |

| I | I2 | I3 | I4 | I5 | I6 | I7 | I8 | I | 9 | I10 | I11 | I12 | I13 | I14 | I15 | I16 | I17 | I18 | I19 |
|---|---|---|---|---|---|---|---|---|---|---|---|---|---|---|---|---|---|---|---|
| I45 | H | COOH | H | H | H | COOH | H | | 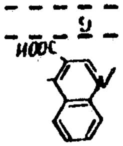 | I,0 | 80,0 | – | – | – | 5,0 | – | – | I4,0 | 7,0 |
| I46 | H | $COOOCH_3$ | H | H | H | $COOCH_3$ | H | | 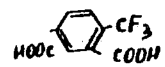 | 5,0 | – | 35,0 | – | – | – | 5,0 | – | 5,0 | I2,0 |
| I47 | H | $CH_3$ | H | $CH_3$ | $CH_3$ | $CH_3$ | H | | 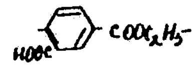 | 0,I | 99,9 | – | – | – | – | – | – | – | 4,0 |
| I48 | H | COOH | H | $CH_3$ | $CH_3$ | COOH | H | | 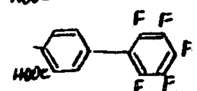 | 0,I | 35,0 | – | – | – | I0,0 | – | – | 4,9 | 3,0 |
| [illegible] | H | $COOC_2H_5$ | H | H | H | $COOC_2H_5$ | H | | 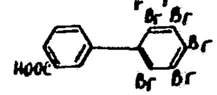 | 0,I | – | 35,0 | – | – | – | – | I0 | 4,9 | 6,5 |
| I50 | H | $CH_3$ | H | H | H | H | H | | 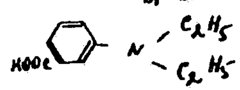 | 0,5 | 75 | – | – | – | 5,0 | – | – | I9,5 | I0,0 |

Anlage zur Tabelle 1

Zusammensetzung der Vitamine im erfindungsgemäßen Mittel, in Gew.-%

| | | |
|---|---|---|
| Zusammensetzung I: | A - Retinol | 1 % |
| | $B_1$ - Thiamin | 6 % |
| | $B_2$ - Riboflavin | 3 % |
| | $B_6$ - Pyridoxin-Chlorid | 6 % |
| | P - Rutin | 6 % |
| | E - Tocopherol-Acetat | 3 % |
| | C - Ascorbinsäure | 60 % |
| | PP - Nikotinsäure | 15 % |
| Zusammensetzung II: | $B_1$ | 5 % |
| | $B_2$ | 5 % |
| | P | 10 % |
| | E | 6 % |
| | C | 54 % |
| | PP | 20 % |
| Zusammensetzung III: | A | 2 % |
| | $B_2$ | 5 % |
| | P | 5 % |
| | E | 4 % |
| | C | 70 % |
| | PP | 14 % |
| Zusammensetzung IV: | $B_1$ | 8 % |
| | $B_2$ | 4 % |
| | P | 10 % |
| | C | 60 % |
| | PP | 18 % |

**Ansprüche**

1. Verwendung von Fluoronderivaten mit Ausnahme des Fluoreszeins und dessen Salzen der allgemeinen Formel I

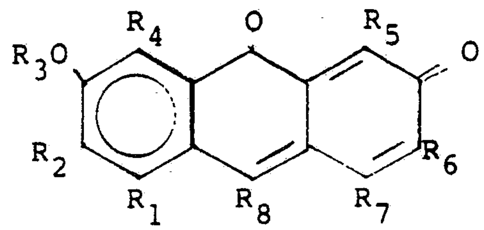

(I)

worin in beliebiger Kombination:

$R_1$ und $R_7$ in beliebiger Kombination Wasserstoff (H), Halogen (F, Cl, Br, J), $J^{131}$, $CH_3$, $CF_3$, $CCl_3$, $C_2H_5$, COOH, $COOCH_3$, $NO_2$, $NH_2$, OH, $OCH_3$, $OC_2H_5$ bedeuten;

$R_2$, $R_4$, $R_5$ und $R_6$ in beliebiger Kombination Wasserstoff (H), Halogen (F, Cl, Br, J), $J^{131}$, sowie die Substituenten, im weiteren als Gruppe A bezeichnet, und zwar aliphatischer $C_1$-$C_5$-Kohlenwasserstoff, linear oder verzweigt, einschließlich Alkyl, Alkenyl, Alkinyl in beliebiger Kombination von Einfach-, Doppel- oder Dreifachbindungen bedeuten; Gruppe B Gruppe A, substituiert durch F, Cl, Br, J, $J^{131}$ von eins bis zur vollständigen Substitution bei beliebiger Kombination unter ihnen bedeutet;

$CH_3$, $CF_3$, $CBr_3$, $CJ_3$, $CJ_3^{131}$, COOH, COOA, COOB, OA, OB, $NO_2$, $NH_2$, eine Substituentengruppe, im weiteren als Gruppe W bezeichnet, Aryl (Phenyl, Naphthyl, Anthryl);

eine Substituentengruppe, im weiteren als Gruppe G bezeichnet, die Gruppe W, substituiert durch F, Cl, Br, J, $J^{131}$, $NO_2$, $NH_2$, OH, $HSO_3$, COOH, $COO^-$, NDE (die Bedeutung von D und E s. unten), $NH-\underset{\overset{\|}{O}}{C}-D$, $-\underset{\overset{\|}{O}}{C}-D$ von eins bis zur vollständigen Substitution in beliebiger Kombination unter ihnen ist, worin die Gruppen, die im weiteren als D- und E-Gruppen bezeichnet werden, A, B, W, G, AB , AG, BW, BG darstellen;

$R_3$ Wasserstoff (H), das Kation eines Alkalimetalls oder Ammonium, A,B, W, G; die Gruppe Q, und zwar Acyl (A-CH=O), die Gruppe Z, und zwar Acyl (B-CH=O) oder Glukuronsäure bedeutet;

$R_8$ Wasserstoff (H), A, B, W, G, AB, AG, BW, BG; die Gruppe I - aliphatischer $C_1$-$C_{15}$-Kohlenwasserstoff, linear oder verzwiegt, einschließlich Alkyl, Alkenyl, Alkinyl in beliebiger Kombination von Einfach-, Doppel- und Dreifachbindungen bedeutet;

eine Gruppe K, - die Gruppe I, substituiert durch F, Cl, Br, J, $J^{131}$, $NO_2$, $NH_2$, OH, $HSO_3$, -COOH, $COO^-$ von eins bis zur vollständigen Substitution in beliebiger Kombination unter ihnen, AI, AK, BI, BK, WI, WK, GI, GK oder die Gruppe L - $H-\underset{\overset{\|}{S}}{C}-NH_2$ ist,

die Gruppe M die Aminosäuren mit $C_1$-$C_5$, LM, Gruppe Y, die einen aromatischen Heterocyclus (Pyridyl, Chinolyl) bezeichnet, die Gruppe V, die die Gruppe Y ist, substituiert durch $NO_2$, $NH_2$, OH, COOH, $COO^-$, $HSO_3^-$, F, Cl, Br, J, $J^{131}$, COOA, COOB,

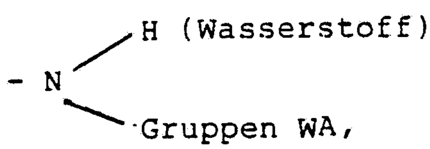

$CH_3$, $CF_3$, $CCl_3$, $CBr_3$, $CJ_3$, $CJ_3^{131}$ von eins bis zur vollständigen Substitution in beliebiger Kombination unter ihnen, AY, BY, AV,BV, WY, GY, GV, WV bedeutet, für eine Röntgen-, Radioisotopen-, Fluoreszenz- und NMR-Kontrastierungen von bösartigen Neubildungen.

2. Verwendung von Fluoronderivaten der allgemeinen Formel I nach Anspruch 1, worin $R_1$ und $R_7$ beide $J^{131}$ bedeuten oder einer $J^{131}$ und der andere beliebig aus: Wasserstoff (H), F, Cl, Br, J, $CH_3$, $CF_3$, $CCl_3$, $C_2H_5$, $COOH_3$, $COOCH_3$, $NO_2$, $NH_2$, OH, $OCH_3$ oder $OC_2H_2$ ist;

$R_2$, $R_4$, $R_5$ und $R_6$ alle $J^{131}$ oder einer oder bis zu drei $J^{131}$ und die anderen eine oder mehrere aus der Gruppe: Wasserstoff (H), F, Cl, Br, J, Gruppen A, B, W, G, AW, AG, BW, BG, $CH_3$, $CF_3$, $CCl_3$, $CBr_3$, $CJ_3$, $CJ_3^{131}$ ,COOH, COOA,COOB, OA, OB, $NO_2$ oder $NH_2$ bedeuten;

$R_3$ Wasserstoff (H), das Kation eines Alkalimetalls oder Ammonium, A, B, W, G, Q, Z oder Glukoronsäure

bedeutet,

$R_8$ H oder $J^{131}$ oder $J^{131}$ bedeutet, welches in beliebiger Kombination in den Gruppen A, B, W, AW, AG, BW, BG, I, K, AI, AK, BI, WI, GI, GK, L, M, LM, Y, V, AY, BY, BV, AV, GY, WV, GY, GV für eine Radioisotopenkontrastierung von bösartigen Neubildungen vorliegen.

3. Verwendung von Fluoronderivaten der allgemeinen Formel I nach Anspruch 2, worin

$R_1$ und $R_7$ jeder aus der Gruppe Wasserstoff (H), $CH_3$, $NO_2$, $NH_2$, OH und $OCH_3$ bedeuten,

$R_2$, $R_4$, $R_5$ und $R_6$ entweder alle $J^{131}$ oder eins bis drei $J^{131}$ und der Rest eins oder mehrere aus der Gruppe Wasserstoff (H), $NO_2$, $NH_2$, $CH_3$, $CCl_3$, $CF_3$, $CJ_3$ und $CJ_3^{131}$ bedeuten;

$R_3$ Wasserstoff (H), das Kation eines Alkalimetalls oder Ammonium bedeutet;

$R_8$ = H oder $J^{131}$ oder $J^{131}$, welches in beliebiger Kombination in der Gruppe W oder G oder V für eine Radioisotopenkontrastierung von bösartigen Neubildungen vorliegen.

4. Verwendung von Fluoronderivaten der allgemeinen Formel I nach Anspruch 1, worin

$R_1$ und $R_7$ beide J oder einer J und der andere Wasserstoff (H), F, Cl, Br, $CH_3$, $CF_3$, $CCl_3$, $C_2H_5$, COOH, $COOCH_3$, $NO_2$, $NH_2$, OH, $OCH_3$ oder $OC_2H_5$ bedeuten;

$R_2$, $R_4$, $R_5$ und $R_6$ alle J oder einer bis drei J und der Rest einen oder mehrere aus der Gruppe Wasserstoff (H), F, Cl, Br, A, B, W, G, AW, AG, BW, BG, $CH_3$, $CF_3$, $CCl_3$, $CBr_3$, COOH, COOA, COOB, OA, OB, $NO_2$, $NH_2$, COOH, COOA, COOB, $NO_2$ und $NH_2$ bedeuten;

$R_3$ Wasserstoff (H), das Kation eines Alkalimetalls, Ammonium, A, B, W, G, Q, Z oder Glukoronsäure bedeutet;

$R_8$ = Wasserstoff (H) oder J oder J, welches in beliebiger Kombination in Gruppen A, B, W, G, AW, AG, BW, BG, I, K, AI, AK, BI, BK, WI, GI, GK, L, M, LM, Y, V, AY, AV, BY, BV, WY, WV, GY und GV vorliegt, für eine Radioisotopenkontrastierung von bösartigen Neubildungen.

5. Verwendung von Fluoronderivaten der allgemeinen Formel I nach Anspruch 4, worin

$R_1$ und $R_7$ aus der Gruppe Wasserstoff (H), $CH_3$, $NO_2$, $NH_2$, OH und $OCH_3$ ausgewählt sind;

$R_2$, $R_4$, $R_5$ und $R_6$ alle J in beliebiger Kombination Wasserstoff (H), F, Cl, $CH_3$, $CF_3$, $CCl_3$, $CJ_3$, $NO_2$ oder $NH_2$ bedeuten;

$R_3$ Wasserstoff (H), das Kation eines Alkalimetalls oder Ammonium bedeutet;

$R_8$ = Wasserstoff (H) oder J oder J, welches in beliebiger Kombination in der Gruppe W oder G oder V für eine Röntgenkontrastierung von bösartigen Neubildungen vorliegt.

6. Verwendung von Fluoronderivaten der allgemeinen Formel I nach Anspruch 1, worin

$R_1$ und $R_7$ F oder einer F und der andere Wasserstoff (H), Cl, Br, J, $CH_3$, $CF_3$, $CCl_3$,$C_2H_5$, COOH, $COOCH_3$, $NO_2$, $NH_2$, OH, $OCH_3$ oder $OC_2H_5$ bedeuten;

$R_2$, $R_4$, $R_5$ und $R_6$ alle F oder eins bis drei F und der Rest ein oder mehrere aus der Gruppe Wasserstoff (H), Cl, Br, J, A, B, W, G, AW, AG, BW, BG, $CH_3$, $CF_3$, $CCl_3$, $CBr_3$, $CJ_3$, COOH, COOA, COOB, OA, OB, $NO_2$ und $NH_2$ bedeuten;

$R_3$ Wasserstoff (H), das Kation eines Alkalimetalls, Ammonium, A, B, W, G, Q, Z oder Glukoronsäure bedeutet;

$R_8$ = Wasserstoff (H) oder F oder F, welches in beliebiger Kombination in der Gruppe A, B, W, G, AW, AG, BW, BG, I, K, AI, BI, BK, AK, WI, WK, GI, GK, L, M, LM, Y , V, AY, BY, BV, AV, WY, GY, WV und GV für eine NMR-Kontrastierung von bösartigen Neubildungen vorliegt.

7. Verwendung von Fluoronderivaten der allgemeinen Formel I nach Anspruch 6, worin

$R_1$ und $R_7$ F oder einer F und der andere Wasserstoff (H), Cl, $CH_3$, $CF_3$, $NO_2$ oder $NH_2$ bedeuten;

$R_2$, $R_4$, $R_5$ und $R_6$ alle F oder einer bis drei F und der Rest ein oder mehrere aus der Gruppe Wasserstoff (H), $CF_3$, $NO_2$, $NH_2$, und OH bedeuten;

$R_3$ Wasserstoff (H), das Kation eines Alkalimetalls oder Ammonium bedeutet;

$R_8$ F oder die Gruppe A, substituiert durch F von eins bis zur vollständigen Substitution, die Gruppe B, substituiert durch F von eins bis zur vollständigen Substitution, die Gruppe I, substituiert durch F von eins bis zur vollständigen Substitution, die Gruppe Y, substituiert durch F, $CF_3$ von eins bis zur vollständigen Substitution, bei beliebiger Kombination unter ihnen, bedeutet, für eine NMR-Kontrastierung von bösartigen Neubildungen.

8. Anwendung von Fluoronderivaten der allgemeinen Formel I nach Anspruch 1, worin

$R_1$ und $R_7$ jeder mindestens eines aus der Gruppe Wasserstoff (H), F, Cl, $CH_3$, $CF_3$, $CCl_3$, COOH, $COOCH_3$, $NO_2$, $NH_2$, OH, $OCH_3$ oder $OC_2H_5$ bedeuten;

$R_2$, $R_4$, $R_5$ und $R_6$ aus der Gruppe Wasserstoff (H), F, Cl, die Gruppe A, sowie die Gruppe A, substituiert durch F, Cl von eins bis zur vollständigen Substitution bei beliebiger Kombination unter ihnen, die durch "P" bezeichnet ist, $CH_3$, $CF_3$, $CCl_3$, COOH, COOA, COOB, OA, OB, $NO_2$, $NH_2$ und W bedeuten;

Gruppe X die Gruppe W, substituiert von eins bis zur vollständigen Substitution durch F, Cl, $NO_2$, $NH_2$, OH, $HSO_3^-$ , COOH oder $COO^-$ ausgewählt sind,

$R_3$ Wasserstoff (H), das Kation eines Alkalimetalls, Ammonium, A, P, W, X, Q oder Acyl (R-CH=O) bedeutet;
$R_8$ = Wasserstoff (H), A, P, W, X, AW, AX, PW, PX und I, Gruppe T die Gruppe I, substituiert durch F, Cl, $NO_2$, $NH_2$, OH, $HSO_3^-$, COOH, $COO^-$, von eins bis zur vollständigen Substituierung bei beliebiger Kombination unter ihnen, AI, PI, AT, PT, WI, XI, WT, XT, L, M, LM, Y, Gruppe U die Gruppe Y, substituiert durch $NO_2$, $NH_2$, OH, COOH, $COO^-$, HS$\overline{O}_3$, F, Cl, COOA, COOP,

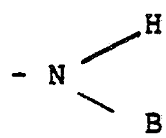 , 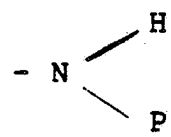 ,

$CH_3$, $CF_3$, $CCl_3$ von eins bis zur vollständigen Substitution in beliebiger Kombination unter ihnen, AY, PY, AU, PU, WY, XU, XY oder WU für eine Fluoreszenzkontrastierung von bösartigen Neubildungen sind.

9. Verwendung von Fluoronderivaten der allgemeinen Formel I nach Anspruch 8, worin
$R_1$ und $R_7$ aus der Gruppe Wasserstoff (H), F, Cl, $NO_2$, $NH_2$ und OH ausgewählt sind;
$R_2$, $R_4$, $R_5$ und $R_6$ aus der Gruppe Wasserstoff (H), F, Cl, $NO_2$, $NH_2$ und OH ausgewählt sind;
$R_3$ Wasserstoff (H), das Kation eines Alkalimetalls oder Ammoniums bedeutet;
$R_8$ Wasserstoff (H), die Gruppe W, X, I, T, Y, oder U für eine Fluoreszenzkontrastierung von bösartigen Neubildungen, bedeutet.

10. Mittel zur Kontrastierung von bösartigen Neubildungen auf der Grundlage von Fluoronderivaten der allgemeinen Formel I nach Anspruch 1, dadurch **gekennzeichnet,** daß die Fluoronderivate zusammen mit Sacchariden und/oder Disacchariden und/oder Vitaminen und/oder Blockatoren der Durchdringungsfähigkeit der Zellmembranen in folgenden Gew.-%:

| | |
|---|---|
| Fluoronderivate | 0,1 bis 89 |
| Saccharide und/oder Disaccharide | 10 bis 98,9 |
| Vitamine | 0,5 bis 85 |
| Blockatoren | 0,5 bis 30 |

vorliegen.

11. Mittel nach Anspruch 10, dadurch **gekennzeichnet,** daß die Fluoronderivate zusammen mit Sacchariden und/oder Disacchariden und/oder Vitaminen und/oder Blockatoren der Durchdringungsfähigkeit der Zellmembranen in folgenden Gew.-%

| | |
|---|---|
| Fluoronderivate | 5 |
| Saccharide und/oder Disaccharide | 90 |
| Vitamine | 2,5 |
| Blockatoren | 2,5 |

vorliegen.

12. Mittel nach Anspruch 10, dadurch **gekennzeichnet,** daß es die folgenden Komponenten in Gew.-%

| | |
|---|---|
| Fluoronderivate | 1 bis 90 |
| Vitamine | 6 bis 96 |
| Blockatoren | 3 bis 93 |

enthält.

13. Mittel nach Anspruch 10 und 12, dadurch **gekennzeichnet,** daß es die folgenden Komponenten in Gew.-%

| | |
|---|---|
| Fluoronderivate | 88 |
| Vitamine | 10 |
| Blockatoren | 2 |

enthält.

14. Mittel nach Anspruch 10, dadurch **gekennzeichnet,** daß es die folgenden Komponenten in Gew.-%

| | |
|---|---|
| Fluoronderivate | 0,1 bis 89 |
| Saccharide oder Disaccharide | 10 bis 98,9 |
| Blockatoren | 1,0 bis 88,9 |

enthält.

15. Mittel nach Anspruch 10 und 14, dadurch **gekennzeichnet,** daß es die folgenden Komponenten in Gew.-%

| | |
|---|---|
| Fluoronderivate | 6 |
| Saccharide oder Disaccharide | 92 |
| Blockatoren | 2 |

enthält.

16. Mittel nach Anspruch 10, dadurch **gekennzeichnet,** daß es die folgenden Komponenten in Gew.-%

| | |
|---|---|
| Fluoronderivate | 0,1 bis 89 |
| Saccharide oder Disaccharide | 10 bis 98,9 |
| Vitamine | 1,0 bis 88,9 |

enthält.

17. Mittel nach Anspruch 10 und 16, dadurch **gekennzeichnet,** daß es die folgenden Komponenten in Gew.-%

| | |
|---|---|
| Fluoronderivate | 8 |
| Saccharide oder Disaccharide | 90 |
| Vitamine | 2 |

enthält.

18. Mittel nach Anspruch 10, dadurch **gekennzeichnet,** daß es die folgenden Komponenten in Gew.-%

| | |
|---|---|
| Fluoronderivate | 0,1 bis 80 |
| Saccharide oder Disaccharide | 20 bis 99,9 |

enthält.

19. Mittel nach Anspruch 10 und 18, dadurch **gekennzeichnet,** daß es die folgenden Komponenten in Gew.-%

| | |
|---|---|
| Fluoronderivate | 10 |
| Saccharide oder Disaccharide | 90 |

enthält.

20. Mittel nach Anspruch 10, dadurch **gekennzeichnet,** daß es die folgenden Komponenten in Gew.-%

| | |
|---|---|
| Fluoronderivate | 20 bis 90 |
| Blockatoren | 10 bis 80 |

enthält.

21. Mittel nach Anspruch 10 und 20, dadurch **gekennzeichnet,** daß es die folgenden Komponenten in Gew.-%

| | |
|---|---|
| Fluoronderivate | 82 |
| Blockatoren | 18 |

enthält.

22. Mittel nach Anspruch 10, dadurch **gekennzeichnet,** daß es die folgenden Komponenten in Gew.-%

| | |
|---|---|
| Fluoronderivate | 1 bis 90 |
| Vitamine | 10 bis 99 |

enthält.

23. Mittel nach Anspruch 10 und 22, dadurch **gekennzeichnet,** daß es die folgenden Komponenten in Gew.-%

| | |
|---|---|
| Fluoronderivate | 82 |
| Vitamine | 18 |

enthält.

24. Mittel nach Anspruch 10, dadurch **gekennzeichnet,** daß es Fluoronderivate in einer Menge von 100 Gew.-% enthält.

25. Mittel nach Anspruch 10, dadurch **gekennzeichnet,** daß als Vitamine die Vitamine der Gruppa A und/oder der Gruppe B und/oder das Vitamin C und/oder die Vitamine P und/oder PP und/oder das Vitamin E enthalten sind.

26. Mittel nach den Ansprüchen 10 bis 25, dadurch **gekennzeichnet,** daß als Vitamine die Vitamine der Gruppe B und/oder die Vitamine C und/oder P und/oder PP und/oder E enthalten sind.

27. Mittel nach Anspruch 10, dadurch **gekennzeichnet,** daß als Saccharide Glykose und/oder Fruk tose enthalten sind.

28. Mittel nach Anspruch 10, dadurch **gekennzeichnet,** daß als Disaccharid Saccharose enthalten ist.

29. Mittel nach Anspruch 10, dadurch **gekennzeichnet,** daß als Blockatoren Antihistaminpräparate, wie 3-Methyl-3-benzyl-1,2,3,4-tetrahydrocarbolin-naphthalin-1,5-disulfanat, Hydrochlorid des 10-(2-Dimethylaminopropyl)-phenothiazins, Hydrochlorid des N-Dimethylaminoethyl-N-(parachlorbenzyl)aminopyridins oder 1-Methyl-2[2-(α-methyl-parachlorbenzhydryloxy)-ethyl]-pyrromedins , 4,9-Dihydro-4-(1-methyl-4-piperidinyliden)-1-OH-benzo[4,5]cyclohepta[1,2]thiophen-10-OH (Hydrofumarat), Hydrochlorid des β-Dimethylaminoethylethers des Benzhydrols oder Hydrochlorids des Chinuklydyl-3(diphenylcarbinols)enthalten sind.

30. Mittel nach Anspruch 1 und 10, dadurch **gekennzeichnet,** daß dieses als Lösung oder Pulver vorliegt.

31. Mittel nach Anspruch 30, dadurch **gekennzeichnet,** daß als Lösungsmittel destilliertes Wasser oder eine 5 bis 30%ige wässerige ethanolische Lösung oder Öllösung Solutio ollosa und vorzugsweise alkoholische Zuckerlösungen Solutio spirituosa saccharum enthalten sind.

Europäisches Patentamt

# EUROPÄISCHER RECHERCHENBERICHT

Nummer der Anmeldung

EP 88 11 7157

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | PATENT ABSTRACTS OF JAPAN, Band 10, Nr. 15 (P-422)[2072], 21. Januar 1986; & JP-A-60 171 436 (MITSUI SEIYAKU KOGYO K.K.) 04-09-1985 * Insgesamt * --- | 1,8,9 | A 61 K 49/00<br>A 61 K 43/00 |
| X | SOVIET INVENTIONS ILLUSTRATED, Woche E27, 8. August 1982, Sektion P/Q, Nr. 56604 E/27, Derwent Publications Ltd, London, GB; & SU-A-792 878 (LENGD NUCLEAR PHYS) 07-02-1982 * Insgesamt * --- | 1,2 | |
| A | US-A-4 256 727 (JOHN W. TRIPLETT et al.) * Spalten 1-2; Tabelle 1, Nr. 4,5,9,13 ; Ansprüche 1,11 * --- | | |
| A | DE-A-1 813 150 (CESKOSLOVENSKA AKADEMIE VED.) * Ansprüche 1,2 * --- | | **RECHERCHIERTE SACHGEBIETE (Int. Cl.4)** |
| A | FR-A- 3 820 (CENTRE NATIONAL DE LA RECHERCHE SCIENTIFIQUE) --- | | A 61 K<br>G 01 N |
| A | US-A-2 704 270 (OLLE GUNNAR ANTON OLSSON) --- | | |
| A | GB-A-1 174 031 (LABORATORIES LABAZ) --- | | |
| T | O.A. NEUMÜLLER.: "RÖMPPS CHEMIE-LEXIKON", 1981, Seite 1336; Franckh'sche Verlagshandlung, Stuttgart, DE * Seite 1336: "Fluorescein" * ----- | | |

Der vorliegende Recherchenbericht wurde für alle Patentansprüche erstellt

| Recherchenort | Abschlußdatum der Recherche | Prüfer |
|---|---|---|
| DEN HAAG | 02-01-1989 | ALVAREZ Y ALVAREZ C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)